# EUROPEAN PATENT SPECIFICATION

(11) **EP 2 313 140 B1**
(45) Date of publication and mention of the grant of the patent: **30.03.2016**
(21) Application number: 09769730.4
(22) Date of filing: 18.06.2009
(51) Int. Cl.: A61M 16/16

(54) **HUMIDIFIER FOR A BREATHING SYSTEM**
BEFEUCHTER FÜR EIN BEATMUNGSSYSTEM
HUMIDIFICATEUR POUR SYSTÈME DE RESPIRATION

(30) Priority: 27.06.2008 US 76250 P
(43) Date of publication of application: 27.04.2011
(73) Proprietor: Koninklijke Philips N.V., 5656 AE Eindhoven (NL)
(72) Inventor: BARCLAY, Mark, W., Briarcliff Manor New York 10510-8001 (US); COLE, Kenneth, E., Briarcliff Manor New York 10510-8001 (US)
(74) Representative: van Velzen, Maaike Mathilde
(86) International application number: PCT/IB2009/052613
(87) International publication number: WO 2009/156921

(56) References cited:
- WO-A-02/066106
- WO-A-2007/019625
- WO-A-2007/038690
- WO-A-2008/024001
- US-A- 3 965 894
- US-B2- 7 111 624

## Description

The present invention relates to humidifiers, for example of the in-line type that are used in breathing systems to humidify gas being provided to a patient.

Various breathing systems use in-line humidifiers to humidify a flow of gas being provided to a patient. For example, positive airway pressure (PAP) machines, such as continuous positive airway pressure (CPAP) device provide a continuous positive airway pressure to a patient via a suitable patient interface (e.g., a mask) for various medical reasons (e.g., to treat sleep apnea). Humidifiers are frequently provided between the PAP machine and the patient interface in order to humidify the otherwise relatively-dry flow of gas generated by the PAP machine. Unfortunately, under certain circumstances (e.g., the humidifier being turned upside-down and/or shaken), liquid (e.g., distilled water) in conventional humidifiers can back-flow or splash from the humidifier into the PAP machine, thereby damaging the PAP machine, interfering with its operation, and/or creating problems associated with the build-up of liquid in the PAP machine. In various PAP machines, even a very small amount of liquid back-flow into the machine can damage a sensor within the machine.

US 3,965,894 discloses a moisture trap inserted lineally in the output line of an oxygen supply apparatus between the regulator and the humidifier. WO 02/066106 discloses a humidifier with structure to prevent backflow of liquid through the humidifier inlet. WO 2008/024001 discloses a respiratory humidifier chamber for use with a breathing assistance apparatus.

WO 2007/019625 discloses a humidifier including a humidifier tub having a heat conducting base plate and a cradle to support the humidifier tub in an operative position. WO 2007/38690 discloses a humidifier with a back-flow prevention valve. US 7,111,624 discloses an apparatus for delivering humidified gases having a connection manifold adapted to connect with inlet and outlet ports of a slide on water chamber in a single slide on motion.

In view of this it is an object to provide an alternative humidifier that prevents a liquid back-flow into the machine.

This object is solved according to the present invention by a humidifier according to claim 1.

One or more embodiments of the present invention provide an in-line humidifier with a liquid trap that discourages liquid from back-flowing through the humidifier's fluid inlet. According to one embodiment of the present invention, the in-line humidifier includes a humidification chamber and a liquid trap. The humidification chamber includes a fluid inlet, a fluid outlet, and a reservoir. The liquid trap includes a chamber, a fluid inlet in fluid communication with the liquid trap chamber, and a fluid outlet in fluid communication with the liquid trap chamber. The fluid outlet of the liquid trap is constructed and arranged to be in fluid communication with the fluid inlet of the humidification chamber. The configuration of the tubings, the chambers, and the tiered arrangement of the humidification chamber and liquid trap provide a series of barriers or obstacles that prevent and/or catch liquid that attempt to flow from the humidifier back into the PAP machine to which the humidifier is connected over a very large range or spatial orientations of the humidifier. That is, the humidifier containing liquid can be moved, turned, oriented, shaken, over a wide range of positions in space with little or none of the liquid exiting the humidification chamber from the gas inlet to the humidifier.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.
FIG. 1 is diagrammatic front view of a breathing system according to an embodiment of the present invention;
FIG. 2 is a left-side perspective view of a humidification chamber and liquid trap of a humidifier thereof;
FIG. 3 is a left-side perspective view of the humidification chamber and liquid trap when detached from each other;
FIG. 4 is a cross-sectional perspective view of the humidification chamber and liquid trap when attached to each other;
FIG. 5 is a cross-sectional view of the humidification chamber and liquid trap when attached to each other;
FIG. 6 is a cross-sectional perspective view of the humidification chamber;
FIG. 7 is a cross-sectional perspective view of the humidification chamber and liquid trap in an upside-down orientation; and
FIG. 8 is a cross-sectional perspective rear view of the humidification chamber and liquid trap when attached to each other.

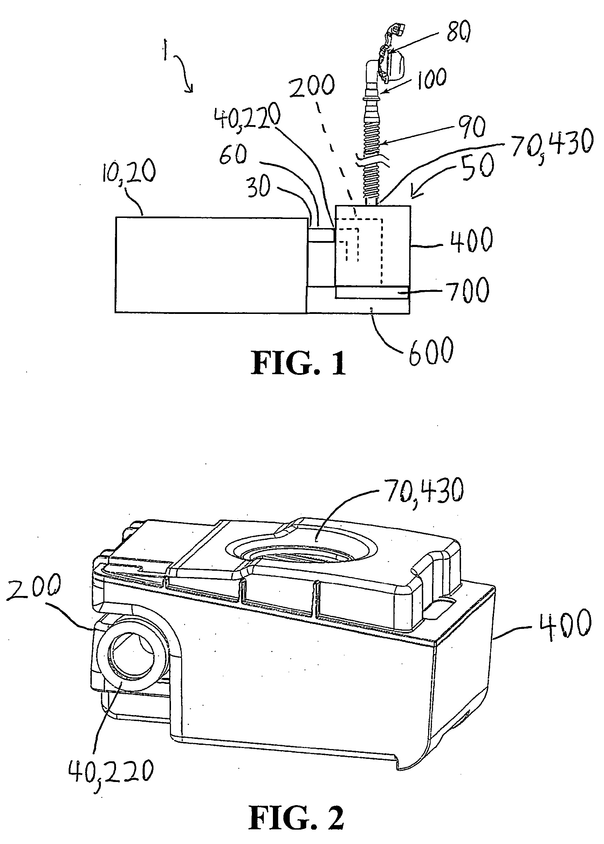

FIGS. 1-8 illustrate a breathing system 1 according to an embodiment of the present invention. The system 1 and its operation are sequentially described below.

As shown in FIG. 1, the system 1 includes a source of gas 10 (also referred to as a pressure generating system), an in-line humidifier 50, and a patient interface 80. Source of gas 10 comprises a PAP machine 20, as is well known in the art (e.g., Respironics' REMstar®, BiPAP®, and SleepEasy® lines of PAP machines). It is be understood that PAP machines, as used herein, refers to any device that generates a flow of gas for delivery to the patient, regardless, for example, of whether flow and/or pressure is constant or varying, the system is invasive or non-invasive, includes a supplement gas such as oxygen, and is single-limb or dual limb. Moreover, according to alternative embodiments of the present invention, a variety of alternative sources of gas 10 may be used as the gas source without deviating from the scope of the present invention (e.g., ventilator, oxygen concentrator, compressed gas tank, etc.) to provide a variety of gases (e.g., air from the ambient environment or a storage container, oxygen-enriched gas, etc.).

A gas outlet 30 of PAP machine 20 fluidly connects to a fluid inlet 40 of humidifier 50 via a suitable passageway (e.g., tubing 60). It should be noted that humidifier 50 can be connected in-line in the gas delivery circuit, which is also referred to as the patient circuit, at any location along that circuit, i.e., humidifier 50 need not be located proximate to the pressure/flow generator or gas source (PAP machine 20).

As shown in FIG. 1, a fluid outlet 70 of humidifier 50 fluidly connects to patient interface 80 via a suitable fluid passageway (e.g., flexible tubing 90). Patient interface 80 may comprise any type of suitable patient interface 80 for directing a flow of gas toward and/or into a patient's airway (e.g., full face mask; nasal mask; total face mask, nasal pillows mask; nasal cannula, intubation tube). In a single-limb pressure support system, which is the type of breathing system shown in FIG. 1, patient interface 80 includes an exhalation port 100 to exhaust a flow of gas to the ambient atmosphere. The present invention contemplates that humidifier 50 can also be used in a dual-limb system that includes an inspiratory limb to deliver a flow of gas to the patient and an expiratory limb to communicate a flow of gas from the patient. In a dual-limb system exhalation port 100 is eliminated.

U.S. Patent Nos. 5,655,522, 6,360,741, 6,401,713, 6,467,477, 6,644,311, and 6,959,710, disclose a variety of pressure generating systems, patient interfaces, and/or other parts of breathing systems that may be used as part of one or more embodiments of the present invention.

Humidifier 50 is described below with reference to FIGS. 1-7. As shown in FIG. 1, humidifier 50 comprises a liquid trap 200, a humidification chamber 400, a base 600, and a heater 700. It should be noted that base 600 and heater 700 are optional. For example, liquid trap 200 and humidification chamber 400 can be coupled to pressure generating system 10 directly and can be used without a heater , i.e., as a passive humidifier.

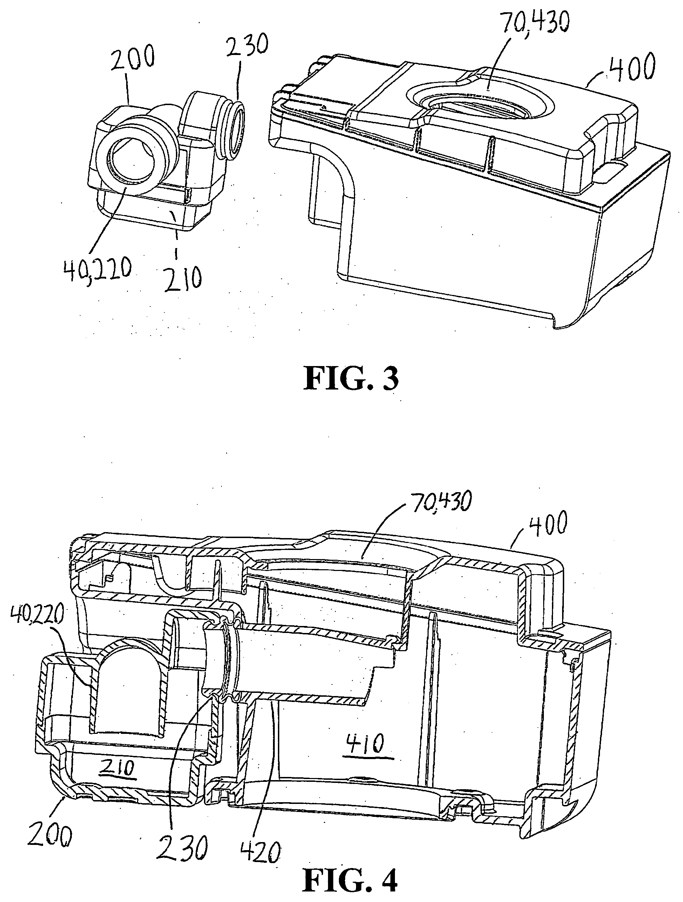

As shown in FIGS. 4, 5, and 8, liquid trap 200 comprises a chamber 210, a fluid inlet 220 that is in fluid communication with chamber 210 and defines fluid inlet 40 of humidifier 50, and a fluid outlet 230 in fluid communication with chamber 210.

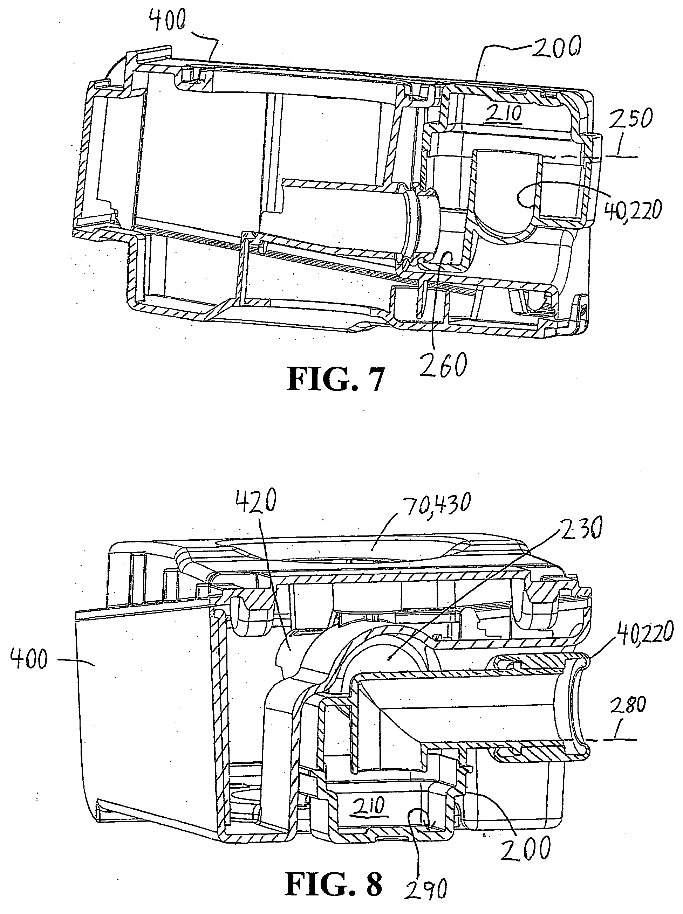

As shown in FIGS. 4, 5, 7, and 8, inlet 220 projects inwardly toward a central portion of chamber 210. Consequently, as shown in FIG. 7, when liquid trap 200 is upside-down, liquid trap's fluid inlet 220 has an upside-down spill height 250 that is elevated relative to a low point 260 of an interior of liquid trap's chamber 210. As used herein, the "spill height" of an opening (e.g., inlet, outlet) means the height of liquid within the associated container (e.g., chamber) at which liquid will begin to spill from the container out through the opening. Consequently, when liquid trap 200 is upside-down, liquid tends not to spill out of the liquid trap through fluid inlet 220 unless a liquid level within liquid trap's chamber 210 rises above upside-down spill height 250.

As shown in FIGS. 4, 5, 7, and 8, fluid inlet 220 extends toward an interior of chamber 210, and in an exemplary embodiment to a central portion of chamber 210, such that the liquid trap's fluid inlet's spill height is elevated relative to a low point of an interior of liquid trap's chamber 210 regardless of an orientation of liquid trap 200. For example, when the liquid trap is upright as shown in FIG. 8, a spill height 280 of inlet 220 is elevated relative to a low point 290 within chamber 210. The same is true when liquid trap 200 is on its sides, front, or back. However, spill height of the inlet 220 may be disposed at the low point of chamber 210 in various orientations without deviating from the scope of the present invention.

As shown in FIG. 8, liquid trap 200 has a minimum trap capacity defined by a volume within the liquid trap's chamber disposed below the spill height of liquid trap's fluid inlet 220 when the liquid trap is in an orientation that minimizes said volume.

Humidification chamber 400 has a reservoir 410, a fluid inlet 420 in fluid communication with the reservoir 410, and a fluid outlet 430 that is in fluid communication with reservoir 410 and defines fluid outlet 70 of humidifier 50. In an exemplary embodiment, gas inlet 420 and the walls of humidification chamber 400 are a unitary structure, e.g., formed as single piece of extruded material.

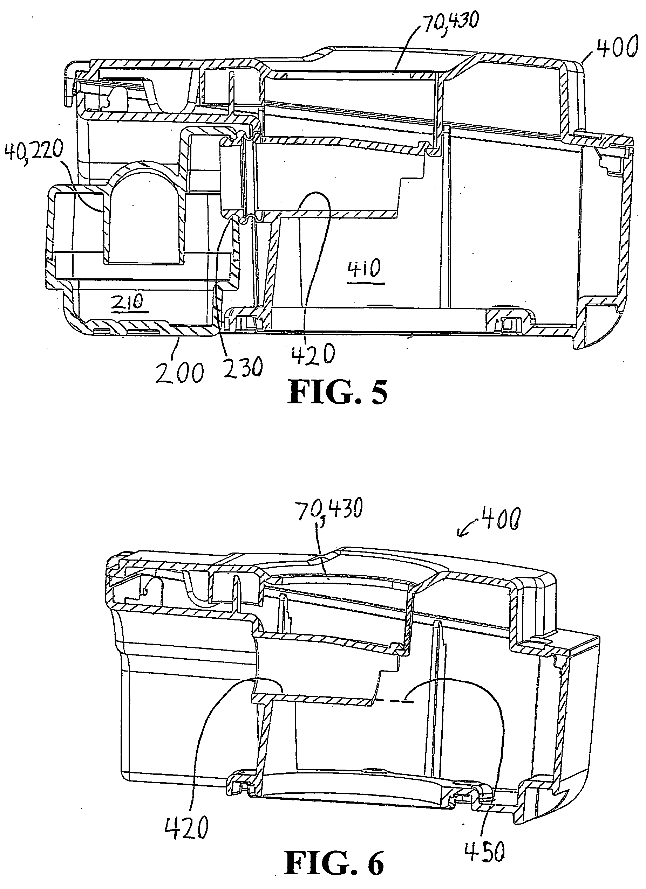

As shown in FIG. 6, fluid inlet 420 of humidification chamber 400 defines an upright spill height 450 such that liquid tends to spill out of humidification chamber 400 through fluid inlet 420 thereof if the humidification chamber is upright and a liquid level within humidification chamber's reservoir 410 rises above spill height 450. Reservoir 410 has a liquid capacity defined by a volume within the interior of humidification chamber 400 disposed below spill height 450 of fluid inlet 420 when humidification chamber 400 is in an upright position.

As shown in FIG. 6, fluid inlet 420 projects into humidification chamber 400 so as the fluid inlet's spill height is elevated relative to a low point of reservoir 410 regardless of an orientation of humidification chamber 400. Such elevation tends to discourage liquid from flowing from humidification chamber 400 to liquid trap 200 when the disposed in non-upright orientations (e.g., sideways, upside-down, etc.). Humidification chamber 400 has a minimum humidification chamber capacity defined by a volume within the humidification chamber disposed below the spill height of the fluid inlet 420 when the humidification chamber is in an orientation that minimizes said volume.

As shown in FIGS. 2-7, humidification chamber 400 and liquid trap 200 are constructed and arranged to be releasably connected to each other (see FIGS. 2, 4, 5, 7) and detached from each other (see FIGS. 3, 6). When humidification chamber 400 and liquid trap 200 are connected to each other, fluid outlet 230 of liquid trap 200 engages and is in fluid communication with fluid inlet 420 of humidification chamber 400. As shown in FIGS. 3 and 4, fluid outlet 230 of liquid trap 200 and fluid inlet 420 of humidification chamber 200 include mating portions that provide at least a partial seal between liquid trap 200 and humidification chamber 400 when they are connected to each other. These same mating portions (or other discrete portions) may also provide a physical connection that tends to keep humidification chamber 400 and liquid trap 200 connected to each other.

As shown in FIG. 1, liquid trap 200 releasably connects to humidifier base 600. However, liquid trap 200 may alternatively be permanently attached to base 600 (e.g., via integral formation) without deviating from the scope of the present invention.

Base 600 releasably attaches to the PAP machine 20. However, base 600 may alternatively be permanently attached to the PAP machine 20 (e.g., via integral formation) without deviating from the scope of the present invention. The attachment (releasable or not) between base 600 and PAP machine 20 facilitates secure connection between the PAP machine's gas outlet 30 and inlet 220 of liquid trap 200 (e.g., via suitable tubing 60). Alternatively, humidifier 50 may comprise a stand-alone unit that connects to the PAP machine 20 via a suitable passageway (e.g., flexible tube) without use of base 600.

When liquid trap 200 is attached to base 600 and humidification chamber 400 is attached to liquid trap 200, humidification chamber 400 also attaches to base 600 such that the base at least partially supports the humidification chamber. Again, it should be understood that the present invention contemplates omitting base 600 in favor of attaching liquid trap 200, humidification chamber 400, or both to the housing of the pressure generating system.

As shown in FIG. 1, heater 700 mounts to base 600 and is positioned relative to reservoir 410 of humidification chamber 400 so as to heat liquid therein to facilitate evaporation of the heated liquid and humidification of the gas passing through humidification chamber 400. Alternatively, heater 700 may be incorporated into humidification chamber 400, itself, without deviating from the scope of the present invention.

If base 600 is attachable to PAP machine 20, the PAP machine and heater 700 may be jointly powered (e.g., via a common electrical plug or battery). According to alternative embodiments of the present invention, heater 700 may be replaced with any other suitable mechanism for enhancing humidification of the gas passing through humidification chamber 400 (e.g., ultrasonic atomizer; humidification via gas entering the chamber underneath the liquid such that resulting bubbles tend to be humidified) or omitted altogether.

Hereinafter, operation of system 1 is described. Prior to use, a user detaches humidification chamber 400 from system 1 and fills it (via fluid outlet 430) to a desired level with a suitable humidification liquid (e.g., distilled water). As shown in FIG. 6, spill height 450 of fluid inlet 420 of humidification chamber 400 tends to prevent overfilling because humidification liquid will spill out through inlet 420 if the liquid exceeds the liquid capacity of humidification chamber 400.

The user then attaches chamber 400 to liquid trap 200 and remainder of system 1 and turns the system on. As shown in FIG. 1, PAP machine 20 directs a flow of gas into humidification chamber 400 via the PAP machine's fluid outlet 30, tubing 60, liquid trap's fluid inlet 40, 220, liquid trap's chamber 210, liquid trap's fluid outlet 230, and humidification chamber's fluid inlet 420. Heater 700 heats the liquid. The liquid evaporates into the gas stream within humidification chamber 400. The gas pressure directs the humidified gas to patient interface 80 via the humidification chamber's fluid outlet 430 and tubing 90, thereby providing a flow of humidified gas to the patient.

If humidifier 50 is inadvertently knocked over or otherwise overturned during use or when otherwise containing liquid (e.g., upside-down as shown in FIG. 7, sideways, etc.), humidification chamber 400 is designed to reduce or eliminate back-flow of the liquid into liquid trap 200. Humidification chamber 400 is configured and sized to prevent liquid from flowing or splashing back through fluid inlet 420 and into liquid trap 200. For example, humidification chamber is provided with an shelf area 411 that will contain fluid in the event the humidification chamber 400 is tilted such that fluid inlet 420 is vertical with the opening of inlet 420 at the lowermost portion of the chamber.

In a worst case scenario (e.g., with outlet 430 plugged and the humidification chamber in an orientation that minimizes its capacity), a certain amount of liquid (e.g., a volume by which the liquid capacity of the humidification chamber 400 exceeds the humidification chamber's minimum humidification chamber capacity) may back-flow into liquid trap 200 via fluid inlet 420 of humidification chamber 400 and fluid outlet 230 of liquid trap 200. The above-discussed spill height and liquid trap capacity of liquid trap 200 helps the trap to trap all or most of such liquid, thereby limiting and/or preventing liquid from further back-flowing into PAP machine 20.

To help ensure that liquid trap 200 has the capacity to trap all or most of the liquid entering it, the liquid trap's minimum trap capacity may exceed a volume by which the liquid capacity of the humidification chamber 400 exceeds the minimum humidification chamber capacity. According to various embodiments of the present invention, the minimum trap capacity is at least 1/20, 1/10, 1/8, 1/5, 1/4, 1/3. 1/2, 2/3, and/or 3/4 as large as the liquid capacity of the humidification chamber 400. According to various embodiments of the present invention, the minimum humidification chamber capacity is at least 1/20, 1/10, 1/8, 1/5, 1/4, 1/3. 1/2, 2/3, and/or 3/4 as large as the liquid capacity of the humidification chamber 400. According to various embodiments of the present invention, the minimum humidification chamber capacity is at least 1/50, 1/30, 1/20, 1/10, 1/8, 1/5, 1/4, 1/3. 1/2, 2/3, and/or 3/4 as large as a total volume within the humidification chamber 400.

According to one or more embodiments of the present invention, the tiered use of back-flow-resisting inlets 220, 430 in liquid trap 200 and humidification chamber 400 provides improved resistance to liquid back-flow into PAP machine 20 under a variety of atypical circumstances (e.g., a combination of one or more of shaking of the system 1 which can cause splashing of the liquid, overturning of the system 1, etc.). Moreover, according to one or more embodiments of present invention, the tiered use of liquid trap 200 and humidification chamber 400 enables an overall reduction in the space required for humidifier 50, while still maintaining a high level of resistance to liquid back-flow.

In short, the configuration of the tubings, the chambers, and the tiered configuration of the humidification chamber and liquid trap provide a series of barriers or obstacles that prevent and/or catch liquid that attempt to flow from the humidifier back into the PAP machine to which the humidifier is connected over a very large range or spatial orientations of the humidifier. That is, the humidifier containing liquid can be moved, turned, oriented, shaken, over a wide range of positions in space with little or none of the liquid exiting the humidification chamber from the gas inlet to the humidifier.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. An in-line humidifier (50) comprising:
a humidification chamber (400) having a fluid inlet (420), a fluid outlet (430), and a reservoir (410);
a liquid trap (200) comprising a liquid trap chamber (210), a fluid inlet (220) in fluid communication with the liquid trap chamber (210), and a fluid outlet (230) in fluid communication with the liquid trap chamber (210), wherein the fluid outlet (230) of the liquid trap (200) is constructed and arranged to be in fluid communication with the fluid inlet (430) of the humidification chamber (400); and
wherein the humidification chamber (400) and the liquid trap (200) are constructed and arranged to be releasably connected to each other, and when the humidification chamber (400) and the liquid trap (200) are connected to each other, the fluid outlet (230) of the liquid trap (200) engages and is in fluid communication with the fluid inlet (420) of the humidification chamber (400)
**characterized in that**,
the fluid inlet (220) of the liquid trap (200) projects inwardly toward a central portion of the liquid trap chamber (210) of the liquid trap (200) and has a liquid trap's fluid inlet's spill height (250, 280) that is elevated relative to a low point (260) of an interior of the liquid trap chamber (210) of the liquid trap (200) regardless of an orientation of the liquid trap (200),
the fluid inlet (420) of the humidification chamber (400) projects into the humidification chamber (400) and has a humidification chamber's fluid inlet's spill height (450) that is elevated relative to a low point of an interior of the humidification chamber (400) regardless of an orientation of the humidification chamber (400), and
wherein the liquid trap's fluid inlet's spill height (250, 280) means the height of liquid with respect to the low point (260) of an interior of the liquid trap chamber (210) of the liquid trap (200) within said liquid trap chamber (210) at which in use a liquid will begin to spill from the liquid trap chamber (210) through the inlet (220), and the humidification chamber's fluid inlet's spill height (450) means the height of liquid with respect to the low point of an interior of the humidification chamber (400) within said humidification chamber (400) at which in use a liquid will begin to spill from the humidification chamber through the inlet (420).

2. The humidifier (50) of claim 1, wherein the fluid outlet (230) of the liquid trap (200) is connected to and in fluid communication with the fluid inlet (420) of the humidification chamber (400).

3. The humidifier (50) of claim 1, wherein the fluid inlet (220) of the liquid trap (200) comprises a fluid inlet (40) of the humidifier, and the fluid outlet (430) of the humidification chamber (400) comprises a fluid outlet (70) of the humidifier (50).

4. The humidifier (50) of claim 1, wherein the fluid inlet (420) of the humidification chamber (400) defines an upright spill height (450) such that liquid tends to spill out of the humidification chamber (400) through the fluid inlet (420) thereof if the humidification chamber (400) is upright and a liquid level within the humidification chamber (400) rises above the upright spill height (450).

5. The humidifier (50) of claim 1, wherein the humidification chamber (400) has a liquid capacity defined by a volume within the humidification chamber (400) disposed below the upright spill height (450) of the fluid inlet (420) of the humidification chamber (400) when the humidification chamber (400) is in an upright position.

6. The humidifier (50) of claim 5, the liquid trap (200) has a non-zero minimum trap capacity defined by a volume within the chamber (210) of the liquid trap (200) disposed below the liquid trap's fluid inlet's spill height when the liquid trap (200) is in an orientation that minimizes said volume.

7. The humidifier (50) of claim 6, wherein the humidification chamber (400) has a minimum capacity defined by a volume within the humidification chamber (400) disposed below a fifth spill height of the fluid inlet (420) of the humidification chamber (400) when the liquid trap (200) is in an orientation that minimizes said volume within the humidification chamber (400).

8. The humidifier (50) of claim 7, wherein the minimum trap capacity and the minimum capacity of the humidification chamber (400) together exceed the liquid capacity of the humidification chamber (400).

9. The humidifier (50) of claim 1, wherein the liquid trap (200) has a minimum trap capacity defined by a volume within the chamber (210) of the liquid trap (200) disposed below the liquid trap's fluid inlet's spill height when the liquid trap (200) is in an orientation that minimizes said volume.

10. The humidifier (50) of claim 1, wherein, when the liquid trap (200) is upside-down:
(a) the fluid inlet (220) of the liquid trap (200) has an upside-down spill height (250) that is elevated relative to the low point (260) of an interior of the chamber of the liquid trap (200), and
(b) liquid in the humidification chamber (400) tends not to spill out of the liquid trap (200) through the fluid inlet (220) thereof unless a liquid level within the chamber (210) of the liquid trap (200) rises above the upside-down spill height.

11. The humidifier (50) of claim 1, further comprising a heater (700) positioned relative to the reservoir (410) to heat liquid disposed in the reservoir (410).

12. A breathing system (1) comprising a humidifier (50) according to any one of claims 1 - 11.

13. A breathing system (1) according to claim 12, further comprising a pressure generating system (10) having a gas outlet (30) connected to the fluid inlet (40) of the humidifier (50).

14. A breathing system (1) according to claim 12 or 13, further comprising a patient interface (80) in fluid communication with the fluid outlet (70) of the humidification chamber (400).

## Patentansprüche

1. In der Linie angeordneter Befeuchter (50), der Folgendes umfasst:
eine Befeuchtungskammer (400) mit einem Fluideinlass (420), einem Fluidauslass (430) und einem Reservoir (410);
eine Flüssigkeitsfalle (200), umfassend eine Flüssigkeitsfallenkammer (210), einen Fluideinlass (220), der in Fluidkommunikation mit der Flüssigkeitsfallenkammer (210) steht, und einen Fluidauslass (230), der in Fluidkommunikation mit der Flüssigkeitsfallenkammer (210) steht, wobei der Fluidauslass (230) der Flüssigkeitsfalle (200) so konstruiert und angeordnet ist, dass er in Fluidkommunikation mit dem Fluideinlass (430) der Befeuchtungskammer (400) steht; und
wobei die Befeuchtungskammer (400) und die Flüssigkeitsfalle (200) so konstruiert und angeordnet sind, dass sie lösbar miteinander verbunden werden können, und der Fluidauslass (230) der Flüssigkeitsfalle (200), wenn die Befeuchtungskammer (400) und die Flüssigkeitsfalls (200) miteinander verbunden sind, in den Fluideinlass (420) der Befeuchtungskammer (400) eingreift und in Fluidkommunikation hiermit steht,
**dadurch gekennzeichnet, dass**
der Fluideinlass (220) der Flüssigkeitsfalls (200) nach innen in Richtung eines zentralen Teils der Flüssigkeitsfallenkammer (210) der Flüssigkeitsfalle (200) ragt und eine Überlaufhöhe (250, 280) des Fluideinlasses der Flüssigkeitsfalle hat, die in Bezug auf einen niedrigen Punkt (260) eines Innenraums der Flüssigkeitsfallenkammer (210) der Flüssigkeitsfalle (200) unabhängig von der Ausrichtung der Flüssigkeitsfalle (200) erhöht ist,
der Fluideinlass (420) der Befeuchtungskammer (400) in die Befeuchtungskammer (400) hineinragt und eine Überlaufhöhe (450) des Fluideinlasses der Befeuchtungskammer hat, die in Bezug auf einen niedrigen Punkt eines Innenraums der Befeuchtungskammer (400) unabhängig von der Ausrichtung der Befeuchtungskammer (400) erhöht ist, und
wobei die Überlaufhöhe (250, 280) des Fluideinlasses der Flüssigkeitsfalle die Höhe der Flüssigkeit in Bezug auf den niedrigen Punkt (260) eines Innenraums der Flüssigkeitsfallenkammer (210) der Flüssigkeitsfalle (200) in der genannten Flüssigkeitsfallenkammer (210) bedeutet, bei der in Betrieb eine Flüssigkeit aus der Flüssigkeitsfallenkammer (210) durch den Einlass (220) überzulaufen beginnt, und wobei die Überlaufhöhe (450) des Fluideinlasses der Befeuchtungskammer die Höhe der Flüssigkeit in Bezug auf den niedrigen Punkt eines Innenraums der Befeuchtungskammer (400) in der genannten Befeuchtungskammer (400) bedeutet, bei der in Betrieb eine Flüssigkeit aus der Befeuchtungskammer durch den Einlass (420) überzulaufen beginnt.

2. Befeuchter (50) nach Anspruch 1, wobei der Fluidauslass (230) der Flüssigkeitsfalle (200) mit dem Fluideinlass (420) der Befeuchtungskammer (400) verbunden ist und in Fluidkommunikation hiermit steht.

3. Befeuchter (50) nach Anspruch 1, wobei der Fluideinlass (220) der Flüssigkeitsfalle (200) einen Fluideinlass (40) des Befeuchters umfasst, und der Fluidauslass (430) der Befeuchtungskammer (400) einen Fluidauslass (70) des Befeuchters (50) umfasst.

4. Befeuchter (50) nach Anspruch 1, wobei der Fluideinlass (420) der Befeuchtungskammer (400) eine aufrechte Überlaufhöhe (450) derartig definiert, dass Flüssigkeit zum Austreten aus der Befeuchtungskammer (400) über deren Fluideinlass (420) neigt, wenn die Befeuchtungskammer (400) aufrecht ist und ein Flüssigkeitsstand in der Befeuchtungskammer (400) über die aufrechte Überlaufhöhe (450) ansteigt.

5. Befeuchter (50) nach Anspruch 1, wobei die Befeuchtungskammer (400) eine Flüssigkeitskapazität hat, die durch ein Volumen in der Befeuchtungskammer (400) unter der aufrechten Überlaufhöhe (450) des Fluideinlasses (420) der Befeuchtungskammer (400) definiert ist, wenn sich die Befeuchtungskammer (400) in einer aufrechten Position befindet.

6. Befeuchter (50) nach Anspruch 5, wobei die Flüssigkeitsfalle (200) eine minimale Auffangkapazität ungleich null hat, die durch ein Volumen in der Kammer (210) der Flüssigkeitsfalle (200) unter der Überlaufhöhe des Fluideinlasses der Flüssigkeitsfalle definiert ist, wenn die Flüssigkeitsfalle (200) eine Ausrichtung hat, die das genannte Volumen minimiert.

7. Befeuchter (50) nach Anspruch 6, wobei die Befeuchtungskammer (400) eine Mindestkapazität hat, die durch ein Volumen in der Befeuchtungskammer (400) unter einer fünften Überlaufhöhe des Fluideinlasses (420) der Befeuchtungskammer (400) definiert ist, wenn die Flüssigkeitsfalle (200) eine Ausrichtung hat, die das genannte Volumen in der Befeuchtungskammer (400) minimiert.

8. Befeuchter (50) nach Anspruch 7, wobei die minimale Auffangkapazität und die minimale Kapazität der Befeuchtungskammer (400) zusammen die Flüssigkeitskapazität der Befeuchtungskammer (400) überschreiten.

9. Befeuchter (50) nach Anspruch 1, wobei die Flüssigkeitsfalle (200) eine minimale Auffangkapazität hat, die durch ein Volumen in der Kammer (210) der Flüssigkeitsfalle (200) unter der Überlaufhöhe des Fluideinlasses der Flüssigkeitsfalle definiert ist, wenn die Flüssigkeitsfalle (200) eine Ausrichtung hat, die das genannte Volumen minimiert.

10. Befeuchter (50) nach Anspruch 1, wobei, wenn die Flüssigkeitsfalle (200) mit der Oberseite nach unten gedreht ist:
(a) der Fluideinlass (220) der Flüssigkeitsfalle (200) eine umgedrehte Überlaufhöhe (250) hat, die in Bezug auf den tiefen Punkt (260) eines Innenraums der Kammer der Flüssigkeitsfalle (200) erhöht ist, und
(b) Flüssigkeit in der Befeuchtungskammer (400) dazu neigt, nicht aus der Flüssigkeitsfalle (200) über deren Fluideinlass (220) auszutreten, wenn nicht ein Flüssigkeitsstand in der Kammer (210) der Flüssigkeitsfalle (200) über die umgedrehte Überlaufhöhe ansteigt.

11. Befeuchter (50) nach Anspruch 1, weiterhin umfassend eine Heizeinheit (700), die in Bezug zum Reservoir (410) angeordnet ist, um die in dem Reservoir (410) befindliche Flüssigkeit zu erwärmen.

12. Beatmungssystem (1) mit einem Befeuchter (50) nach einem der Ansprüche 1 bis 11.

13. Beatmungssystem (1) nach Anspruch 12, weiterhin umfassend ein Druckerzeugungssystem (10) mit einem Gasauslass (30), der mit dem Fluideinlass (40) des Befeuchters (50) verbunden ist.

14. Beatmungssystem (1) nach Anspruch 12 oder 13, weiterhin umfassend eine Patientenschnittstelle (80) in Fluidkommunikation mit dem Fluidauslass (70) der Befeuchtungskammer (400).

## Revendications

1. Humidificateur en ligne (50) comprenant :
une chambre d'humidification (400) ayant une entrée de fluide (420), une sortie de fluide (430) et un réservoir (410) ;
un piège à liquide (200) comprenant une chambre de piège à liquide (210), une entré de fluide (220) en communication fluidique avec la chambre de piège à liquide (210) et une sortie de fluide (230) en communication fluidique avec la chambre de piège à liquide (210), dans lequel la sortie de fluide (230) du piège à liquide (200) est conçue et aménagée pour être en communication fluidique avec l'entrée de fluide (430) de la chambre d'humidification (400) ; et
dans lequel la chambre d'humidification (400) et le piège à liquide (200) sont conçus et aménagés pour être raccordés l'un à l'autre de manière amovible et, lorsque la chambre d'humidification (400) et le piège à liquide (200) sont raccordés l'un à l'autre, la sortie de fluide (230) du piège à liquide (200) s'engage en communication fluidique avec l'entrée de fluide (420) de la chambre d'humidification (400),
**caractérisé en ce que** :
l'entrée de fluide (220) du piège à liquide (200) fait saillie vers l'intérieur en direction d'une partie centrale de la chambre (210) du piège à liquide (200) et a une hauteur de déversement d'entrée de fluide (250, 280) du piège à liquide qui est élevée par rapport à un point bas (260) de l'intérieur de la chambre (210) du piège à liquide (200) quelle que soit l'orientation du piège à liquide (200),
l'entrée de fluide (420) de la chambre d'humidification (400) fait saillie dans la chambre d'humidification (400) et présente une hauteur de déversement d'entrée de fluide (450) de la chambre d'humidification qui est élevée par rapport à un point bas de l'intérieur de la chambre d'humidification (400) quelle que soit l'orientation de la chambre d'humidification (400), et
dans lequel la hauteur de déversement d'entrée de fluide (250, 280) du piège à liquide désigne la hauteur de liquide par rapport au point bas (260) de l'intérieur de la chambre (210) du piège à liquide (200) dans ladite chambre de piège à liquide (210) où, en service, un liquide commencera à se déverser de la chambre (210) du piège à liquide à travers l'entrée (220) et la hauteur de déversement (450) de l'entrée de fluide de la chambre d'humidification désigne la hauteur du liquide par rapport au point bas de l'intérieur de la chambre d'humidification (400) au sein de ladite chambre d'humidification (400) où, en service, un liquide commencera à se déverser de la chambre d'humidification à travers l'entrée (420).

2. Humidificateur (50) selon la revendication 1, dans lequel la sortie de fluide (230) du piège à liquide (200) est raccordée en communication fluidique avec l'entrée de fluide (420) de la chambre d'humidification (400).

3. Humidificateur (50) selon la revendication 1, dans lequel l'entrée de fluide (220) du piège à liquide (200) comprend une entrée de fluide (40) de l'humidificateur et la sortie de fluide (430) de la chambre d'humidification (400) comprend une sortie de fluide (70) de l'humidificateur (50).

4. Humidificateur (50) selon la revendication 1, dans lequel l'entrée de fluide (420) de la chambre d'humidification (400) définit une hauteur de déversement verticale (450) telle que le liquide tende à se déverser de la chambre d'humidification (400) à travers son entrée de fluide (420) si la chambre d'humidification (400) est verticale et que le niveau de liquide dans la chambre d'humidification (400) s'élève au-dessus de la hauteur de déversement verticale (450).

5. Humidificateur (50) selon la revendication 1, dans lequel la chambre d'humidification (400) a une capacité de liquide définie par un volume dans la chambre d'humidification (400) disposé en dessous de la hauteur de déversement verticale (450) de l'entrée de fluide (420) de la chambre d'humidification (400) lorsque la chambre d'humidification (400) est en position verticale.

6. Humidificateur (50) selon la revendication 5, dans lequel le piège à liquide (200) a une capacité de piégeage minimale non nulle définie par un volume dans la chambre (210) du piège à liquide (200) disposé en dessous de la hauteur de déversement de l'entrée de fluide du piège à liquide lorsque le piège à liquide (200) se trouve dans une orientation qui minimise ledit volume.

7. Humidificateur (50) selon la revendication 6, dans lequel la chambre d'humidification (400) a une capacité minimale définie par un volume dans la chambre d'humidification (400) disposé en dessous d'une cinquième hauteur de déversement de l'entrée de fluide (420) de la chambre d'humidification (400) lorsque le piège à liquide (200) se trouve dans une orientation qui minimise ledit volume dans la chambre d'humidification (400).

8. Humidificateur (50) selon la revendication 7, dans lequel la capacité minimale du piège et la capacité minimale de la chambre d'humidification (400) dépassent conjointement la capacité de liquide de la chambre d'humidification (400).

9. Humidificateur (50) selon la revendication 1, dans lequel le piège à liquide (200) a une capacité de piégeage minimale défini par un volume dans la chambre (210) du piège à liquide (200) disposé en dessous de la hauteur de déversement de l'entrée de fluide du piège à liquide lorsque le piège à liquide 200) se trouve dans une orientation qui minimise ledit volume.

10. Humidificateur (50) selon la revendication 1, dans lequel, lorsque le piège à liquide (200) est à l'envers :
(a) l'entrée de fluide (220) du piège à liquide (200) a une hauteur de déversement à l'envers (250) qui est élevée par rapport au point bas (260) de l'intérieur de la chambre du piège à liquide (200) et
(b) le liquide dans la chambre d'humidification (400) a tendance à ne pas se déverser du piège à liquide (200) à travers son entrée de fluide (220) à moins qu'un niveau de liquide à l'intérieur de la chambre (210) du piège à liquide (200) ne s'élève au-dessus de la hauteur de déversement à l'envers.

11. Humidificateur (50) selon la revendication 1, comprenant en outre un dispositif de chauffage (700) positionné par rapport au réservoir (410) pour chauffer le liquide disposé dans le réservoir (410).

12. Système respiratoire (1) comprenant un humidificateur (50) selon l'une quelconque des revendications 1 à 11.

13. Système respiratoire (1) selon la revendication 12, comprenant en outre un système générateur de pression (10) ayant une sortie de gaz (30) raccordée à l'entrée de fluide (40) de l'humidificateur (50).

14. Système respiratoire (1) selon la revendication 12 ou la revendication 13, comprenant en outre une interface de patient (80) en communication fluidique avec la sortie de fluide (70) de la chambre d'humidification (400).
